# EUROPEAN PATENT APPLICATION

(11) **EP 4 541 895 A1**
(43) Date of publication of application: **23.04.2025**
(21) Application number: 23823984.2
(22) Date of filing: 15.06.2023
(51) Int. Cl.: C12N 15/115, C12Q 1/68, G01N 33/543, G01N 33/553, G01N 33/569

(54) **METHOD FOR DETECTING ANALYTE USING POLYNUCLEOTIDE**

(30) Priority: 15.06.2022 JP 2022096870
(71) Applicant: National University Corporation Tokyo University Of Agriculture and Technology, Fuchu-shi Tokyo 183-8538 (JP); JNC Corporation, Chiyoda-ku Tokyo 100-8105 (JP)
(72) Inventor: IKEBUKURO, Kazunori, Fuchu-shi, Tokyo 183-8538 (JP); SAITO, Hiroshi, Yokohama-shi, Kanagawa 236-8605 (JP); OHNISHI, Noriyuki, Tokyo 100-8105 (JP)
(74) Representative: Becker, Eberhard
(86) International application number: PCT/JP2023/022324
(87) International publication number: WO 2023/243694

(57) **Abstract**

The present invention pertains to a method for detecting an analyte contained in a sample, the method including: (1) mixing the sample and salt with a gold-nanoparticle-labeled aptamer in which gold nanoparticles are bound to an aptamer capable of binding to the analyte; and (2) judging that the sample contains the analyte when there is no aggregation of gold-nanoparticle-labeled aptamer in the mixture obtained by (1).

## Description

### Technical Field

The present invention relates to a method for detecting an analyte using a polynucleotide.

### Related Art

Currently, the measurement of analytes such as proteins in the specimen is mainly carried out by immunoassay methods. There are various immunoassay methods in practical use, all of which use an antibody specific to the analyte. Although specific antibodies against an analyte may be produced by standard methods, these methods are time-consuming and expensive, which is one of the factors that increase the cost of various immunoassay methods. Furthermore, methods that use enzyme-substrate reactions, such as ELISA and CLEIA, are also widely used. However, these methods require many special reagents, such as luminescent reagents, in addition to antibodies, and involve multiple steps, such as incubating the specimen and each reagent, washing, and measuring luminescence, making the operations complicated. Moreover, each step takes an extremely long time, and the work costs are considered high.

On the other hand, it is known to those skilled in the art that aptamers, which are polynucleotide molecules that specifically bind to any molecule, may be used in the same way as antibodies. Nucleic acid aptamers, such as DNA aptamers and RNA aptamers, are expected to be candidates for new pharmaceuticals and diagnostic agents because they have characteristics that antibodies do not have, such as being inexpensive to produce through chemical synthesis, being able to be stored at room temperature in a dry state, and being easy to design, e.g., their binding ability to the analyte may be controlled.

However, since the end of 2019, the novel coronavirus (hereinafter also referred to as "SARS-CoV-2") has been spreading. SARS-CoV-2 is known to infect human respiratory epithelial cells using the same receptor, angiotensin-converting enzyme II (hereinafter also referred to as "ACE2"), as SARS-CoV (Non-Patent Documents 1 to 3). In the early stages of SARS-CoV-2 infection, the receptor-binding domain (hereinafter also referred to as "RBD") of the SARS-CoV-2 spike protein (hereinafter also referred to as "S protein") interacts with ACE2 expressed on host cells. The function of the RBD in the coronavirus S protein is important and affects host range, viral specificity, mortality, etc. For this reason, the RBD of the SARS-CoV-2 S protein is expected to be an important target for the diagnosis, treatment, and vaccine of SARS-CoV-2 (Non-Patent Document 4).

Aptamers (diameter: approximately 2-3 nm) are smaller in size than antibodies (diameter: approximately 12-15 nm), and therefore are less likely to cause steric hindrance on the surface of coronaviruses (diameter: approximately 100 nm). Under these circumstances, an aptamer capable of detecting SARS-CoV-2 has been reported (Non-Patent Document 4).

Furthermore, a known method for detecting an analyte is the colloidal gold aggregation method (also called "colloidal gold colorimetry"), which uses the aggregation of gold nanoparticles as an indicator. The colloidal gold aggregation method uses gold nanoparticle-labeled antibodies as detection reagents and recognizes the presence of an antigen when gold nanoparticles aggregate.

### Prior-Art Document

### Non-Patent Document

Non-Patent Document 1: Daniel Wrapp, et al., "Cryo-EM structure of the 2019-nCoV spike in the prefusion conformation", Science 2020, 367, 1260-1263.
Non-Patent Document 2: Alexandra C Walls, et al., "Structure, Function, and Antigenicity of the SARS-CoV-2 Spike Glycoprotein", Cell 2020, 181, 281-292.
Non-Patent Document 3: Renhong Yan, et al., "Structural basis for the recognition of SARS-CoV-2 by full-length human ACE2", Science 2020, 367, 1444-1448.
Non-Patent Document 4: Yanling Song, et al., "Discovery of Aptamers Targeting the Receptor-Binding Domain of the SARS-CoV-2 Spike Glycoprotein", Analytical Chemistry, 2020, 92, 14, 9895-9900.

### SUMMARY OF INVETION

### Problems to be solved by the Invention

The present invention has been made in view of the above circumstances, and an object of the present invention is to provide a method for detecting an analyte quickly, inexpensively, simply and with high accuracy.

### Means for solving the Problems

The inventors have discovered that, unlike conventional colloidal gold aggregation methods, by mixing a sample and salt with a gold-nanoparticle-labeled aptamer in which gold nanoparticles are bound to an aptamer capable of binding to an analyte, it is possible to judge that the sample contains the analyte when there is no aggregation of gold-nanoparticle-labeled aptamer.

That is, the present invention includes the following embodiments.
1. A method for detecting an analyte contained in a sample includes the following (1) and (2):
   (1) The sample and salt are mixed with a gold-nanoparticle-labeled aptamer in which gold nanoparticles are bound to an aptamer capable of binding to the analyte.
   (2) It is judged that the sample contains the analyte when there is no aggregation of the gold-nanoparticle-labeled aptamer in a mixture obtained in (1).
2. The method according to 1 above, in which the salt is sodium chloride.
3. The method according to 1 or 2 above, in which the aptamer is a polynucleotide consisting of a base sequence shown in SEQ ID NO:1.
4. The method according to 3 above, in which the analyte is a SARS-CoV-2 spike protein.

### Effects of Invention

According to the detection method of one embodiment of the present invention, an analyte can be detected simply and quickly.

### BRIEF DESCRIPTION OF DRAWINGS

FIG. 1 is a schematic diagram showing the principle of a detection method according to one embodiment of the present invention.
FIG. 2 is a schematic diagram showing the principle of aptamer blotting in Reference Example 1.
FIG. 3 shows the results of aptamer blotting using various aptamers.
FIG. 4 shows the results of aptamer blotting using RBD-46 as the aptamer.
FIG. 5A shows a graph in which the horizontal axis represents the aptamer concentration in an aptamer solution, and the vertical axis represents the chemiluminescence intensity.
FIG. 5B shows a graph in which the horizontal axis represents the aptamer concentration in the aptamer solution, and the vertical axis represents the value obtained by dividing the aptamer concentration in the aptamer solution by the chemiluminescence intensity.

### DESCRIPTION OF THE EMBODIMENTS

The detection method according to one embodiment of the present invention will be described in detail below, but this is merely one example of a preferred embodiment.

One embodiment of the present invention is a method for detecting an analyte in a sample, including the following steps (1) and (2):
(1) The sample and salt are mixed with a gold-nanoparticle-labeled aptamer in which gold nanoparticles are bound to an aptamer capable of binding to an analyte.
(2) It is judged that the sample contains the analyte when there is no aggregation of gold-nanoparticle-labeled aptamer in the mixture obtained in (1).

### Each step will be described below.

### <(1) Mix a sample and salt with a gold-nanoparticle-labeled aptamer in which gold nanoparticles are bound to an aptamer capable of binding to an analyte. >

Step (1) is a step of mixing a sample and salt with a gold-nanoparticle-labeled aptamer in which gold nanoparticles are bound to an aptamer capable of binding to an analyte. In step (1), it is preferable to first prepare a solution (a) containing a gold-nanoparticle-labeled aptamer and a solution (b) containing a sample and salt, and then mix the solution (a) with the solution (b). By preparing solution (a) and solution (b) in advance, it is possible to suppress aggregation of the gold-nanoparticle-labeled aptamer in the presence of salt when the analyte is contained.

### (Sample and Analyte)

The sample includes substances used in clinical diagnosis. For example, the sample includes body fluids, urine, sputum, feces, saliva, and the like. Examples of analytes contained in the sample include the novel coronavirus (SARS-CoV-2), SARS-CoV-2 spike protein, influenza virus, vascular endothelial growth factor (VEGF), thrombin, human immunoglobulin G, human immunoglobulin M, human immunoglobulin A, human immunoglobulin E, human albumin, human fibrinogen (fibrin and its degradation products), alpha-fetoprotein (AFP), C-reactive protein (CRP), myoglobin, carcinoembryonic antigen, hepatitis virus antigen, human chorionic gonadotropin (hCG), human placental lactogen (HPL), HIV virus antigen, allergens, bacterial toxins, bacterial antigens, enzymes, hormones (e.g., human thyroid stimulating hormone (TSH), insulin, etc.), and drugs. Other examples of the sample include secretions collected from any part of the body, and analytes include causative substances related to any disease, such as biomarkers. These samples often contain many different types and large amounts of contaminants, but it is not always necessary to carry out a preliminary step of removing the contaminants before measurement.

For example, when a polynucleotide consisting of the base sequence shown in SEQ ID NO: 1 is used as an aptamer, the analyte may be SARS-CoV-2, particularly the SARS-CoV-2 spike protein.

### (Aptamer)

The aptamer may be an aptamer that recognizes a specific site of the analyte, or may be a mixture of aptamers that recognize different sites of the analyte, as long as it is capable of binding to the analyte. The aptamer in the present invention may be of any type, and examples thereof include DNA aptamers and RNA aptamers that form a hairpin-loop or G-quadruplex structure. Also, for example, the aptamer may be of a bivalent type in which two molecules of an aptamer that recognize a specific site on an analyte are linked by a linker, or of a bispecific type in which two types of aptamers that recognize different sites on an analyte are linked by a linker. A mixture of aptamers recognizing different sites on an analyte may be used, for example, in sandwich assays or multi-detection.

Aptamer is used to specifically recognize an analyte. Compared with the use of antibodies, for example, DNA aptamers may be easily modified in various ways, such as by labeling with biotin at the 5' end and 3' end, and retain a high degree of binding ability to the antigen even after such processing. Furthermore, since it is easy to design a DNA aptamer that has high binding ability and specificity to an antigen (a dissociation constant lower than that of an antibody), detection can be achieved with high sensitivity in a short period of time.

An example of an aptamer is a polynucleotide consisting of the base sequence shown in SEQ ID NO:1. A polynucleotide consisting of the base sequence shown in SEQ ID NO:1 refers to a polynucleotide consisting only of the base sequence shown in SEQ ID NO:1 and containing no other base sequences. Since the polynucleotide consists of the base sequence shown in SEQ ID NO:1, it exhibits high affinity for SARS-CoV-2.

The dissociation constant of an aptamer may be measured by well-known methods. The dissociation constant of the polynucleotide consisting of the base sequence shown in SEQ ID NO: 1 to the receptor-binding domain of the SARS-CoV-2 S protein is 34.7 nM, as measured by the methods described in Reference Examples 1 and 2 below.

The polynucleotide consisting of the base sequence shown in SEQ ID NO:1 may be easily modified in various ways, such as by labeling with biotin at the 5' end and 3' end, and retain a high degree of binding ability to the RBD of the SARS-CoV-2 S protein even after such processing. Moreover, since the polynucleotide consisting of the base sequence shown in SEQ ID NO:1 has high binding ability and specificity to the RBD of the SARS-CoV-2 S protein, SARS-CoV-2 in a sample can be detected with high sensitivity in a short period of time.

The polynucleotide consisting of the base sequence shown in SEQ ID NO:1 can be easily chemically synthesized using a commercially available nucleic acid synthesizer since its base sequence is clear.

### (Gold Nanoparticles)

The gold nanoparticles used in the method of the present invention may be commercially available, or may be prepared by a method commonly used by those skilled in the art (e.g., a method of reducing chloroauric acid with sodium citrate). The color of the colloidal gold varies depending on factors such as the particle size of the gold nanoparticles contained therein, but in one embodiment of the detection method of the present invention, red is preferred from the standpoint of detection sensitivity and accuracy of the analyte. The particle size of the gold nanoparticles is preferably 10 to 100 nm, more preferably 20 to 80 nm, and particularly preferably 40 to 80 nm. The gold nanoparticles having a particle size within the above range have the advantage of excellent visibility of red color and easy visual determination based on color tone change. The shape of the gold nanoparticles contained is not particularly limited, and examples thereof include various shapes such as spheres, rods, plates, and sea urchins.

### (Binding of gold nanoparticles to aptamer)

The method for binding an aptamer to gold nanoparticles is not particularly limited, but examples include a method of binding a biotin-labeled aptamer or an amino group-introduced aptamer to gold nanoparticles using a passive adsorption method based on electrostatic and hydrophobic interactions that occur between the surface layers of protein and gold nanoparticle, and a method of binding substances that have affinity for each other (e.g., biotin and streptavidin or avidin) to both the aptamer side and the gold nanoparticle side, and binding the aptamer and gold nanoparticles via these substances. From the viewpoint of gold nanoparticle aggregation, the pH during the reaction is preferably 8 to 10.

Specifically, for example, at least one of avidin and streptavidin is bound to gold nanoparticles by a passive adsorption method based on electrostatic and hydrophobic interactions that occur between the surface layers of protein and gold nanoparticle. Furthermore, labeling of the aptamer with biotin or the like is carried out according to a conventional method, and the aptamer is labeled with biotin at the 5' end or 3' end during oligo DNA synthesis. When a biotin-labeled aptamer is mixed with avidin- or streptavidin-immobilized gold nanoparticles, the aptamer and the gold nanoparticles are bound together via the bond between biotin and avidin or streptavidin.

In order to suppress non-specific adsorption to the gold nanoparticles, it is preferable to block the gold nanoparticles after binding the gold nanoparticles to the aptamer. For blocking, it is preferable to use BSA (bovine serum albumin) from the viewpoint similar to that of immunoassays utilizing antigen-antibody reactions, such as immunochromatography, ELISA, and Western blotting.

### (Salt)

The salt may be any salt, for example, sodium chloride.

The salt is preferably contained in an amount of 125 to 250 mM relative to the sample-containing solution. For example, when a solution containing a gold-nanoparticle-labeled aptamer serving as a detection reagent is mixed with a solution containing a sample and salt at a 1:1 (volume ratio), it is more preferable to prepare the mixture such that the final concentration of the salt is 62.5 to 125 mM.

### <(2) judge that the sample contains the analyte when there is no aggregation of gold-nanoparticle-labeled aptamer in the mixture obtained in (1) >

Step (2) is a step of judging whether or not the sample contains an analyte based on the degree of aggregation of the gold-nanoparticle-labeled aptamer in the mixture obtained in step (1). The above (1) refers to the process described in the third paragraph in [DESCRIPTION OF THE EMBODIMENTS] to the paragraph immediately preceding this paragraph.

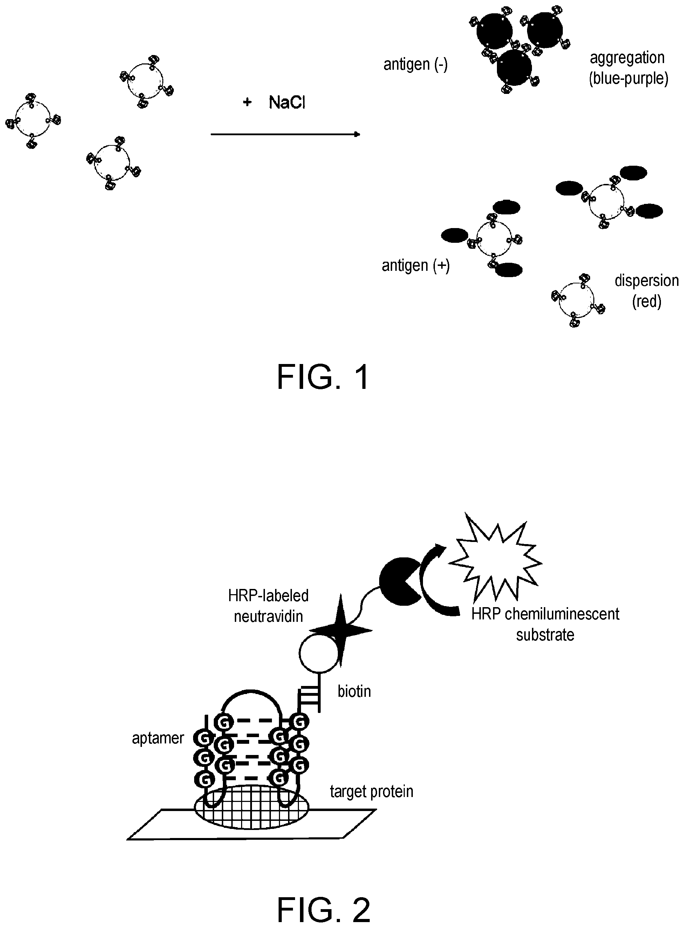

A schematic diagram of the principle of the detection method according to one embodiment of the present invention is shown in FIG. 1. In the absence of the analyte, the gold-nanoparticle-labeled aptamers aggregate. When the gold-nanoparticle-labeled aptamers aggregate in the mixed solution, the solution becomes colorless or blue-purple. It is presumed that in the absence of the analyte, the higher-order structure of the aptamer is changed by the salt in the solution, and various intermolecular interactions are formed between the aptamers, resulting in aggregation of the gold-nanoparticle-labeled aptamers.

In the presence of the analyte, there is no aggregation of gold-nanoparticle-labeled aptamer and dispersion thereof is maintained. When the gold-nanoparticle-labeled aptamer is dispersed in the mixed solution, the solution turns a reddish color, such as red or light red. It is presumed that when the analyte is present, the analyte and the aptamer bind to each other, such that the higher-order structure of the aptamer is not affected by the salt and the dispersion of the gold-nanoparticle-labeled aptamer is maintained.

The degree of aggregation, that is, the presence or absence of dispersion, may be determined, for example, by visual observation, turbidity measurement, absorbance measurement, etc., and is preferably determined by visual observation. Visual inspection, turbidity measurement, absorbance measurement, etc. may be carried out intermittently at a certain time point, or may be carried out continuously over time. The determination may also be made based on the difference between a turbidity measurement value at a certain point in time and a turbidity measurement value at another point in time. By measuring the turbidity, the concentration of the analyte may be quantified.

The above procedure is achieved by using a sample, a gold-nanoparticle-labeled aptamer, and salt, and is performed without using any special reagents or equipment, and is therefore inexpensive and simple. Furthermore, the presence or absence of the analyte can be rapidly judged simply by visually observing the degree of aggregation.

### Example

The present invention will be specifically described below by way of examples, but the present invention is not limited to the following examples as long as it does not depart from the gist of the present invention.

In Reference Examples 1 and 2, the novel coronavirus-binding aptamer was evaluated. Then, using the novel coronavirus-binding aptamers evaluated in Reference Examples 1 and 2, a detection method using gold nanoparticles was evaluated in Example 1.

### [Reference Example 1] Comparison of novel coronavirus-binding aptamer

Prior to aptamer blotting, the following materials and specimens were prepared.

### (material)

A nitrocellulose membrane, fetal bovine serum (BSA), HRP-labeled neutravidin, and an HRP chemiluminescent substrate were prepared. All other chemical reagents were of analytical grade.

### (PBS)

PBS (pH 7.4) containing 8.1 mM Na₂HPO₄, 1.4 mM KH₂PO₄, 137 mM NaCl, 2.7 mM KCl was prepared.

### (Preparation of SARS-CoV-2 S protein specimen)

SARS-CoV-2 (2019-nCoV) Spike RBD Recombinant protein (manufactured by Sino Biological Co., Ltd.) was prepared as a SARS-CoV-2 S protein specimen.

### (Preparation of aptamer)

Each aptamer having the base sequence shown in Table 1 below was synthesized and modified at the 5' end with biotin.

[Table 1]

**Table 1**

| Name | SEQ ID NO |
|---|---|
| RBD-1C | SEQ ID NO: 2 |
| RBD-4C | SEQ ID NO: 3 |
| RBD-41 | SEQ ID NO: 4 |
| RBD-42 | SEQ ID NO: 5 |
| RBD-43 | SEQ ID NO: 6 |
| RBD-44 | SEQ ID NO: 7 |
| RBD-45 | SEQ ID NO: 8 |
| RBD-46 | SEQ ID NO: 1 |
| RBD-47 | SEQ ID NO: 9 |
| RBD-48 | SEQ ID NO: 10 |

Aptamer blotting was performed according to the following procedure. The principle of aptamer blotting is shown in FIG. 2.

### (1) Application of target protein specimen

A specimen of SARS-CoV-2 S protein fused with 1 pmol/L mouse IgG Fc and 1 pmol/L mouse IgG Fc were each applied to different locations on a 50 mm x 15 mm nitrocellulose membrane by 2 pmol each, following by 1 hour of blocking with PBS containing 2% BSA for 1 hour, and 5 minutes of repeated washing with PBS for three times. Since the S protein is a membrane protein and difficult to express, it was expressed by fusion with mouse IgG Fc.

### (2) Aptamer reaction

1 ml of a solution obtained by dissolving an aptamer modified with biotin at the 5' end in PBS (hereinafter also referred to as "aptamer solution") was dropped onto the membrane to a final concentration of 500 nmol/L, and incubated at room temperature for 1 hour, following by 5 minutes of repeated washing with PBS for three times.

### (3) Reaction of HRP-labeled neutravidin

l ml of HRP-labeled neutravidin was dropped onto the membrane and incubated at room temperature for 1 hour, following by 5 minutes of repeated washing with PBS for three times.

### (4) Reaction of HRP chemiluminescent substrate

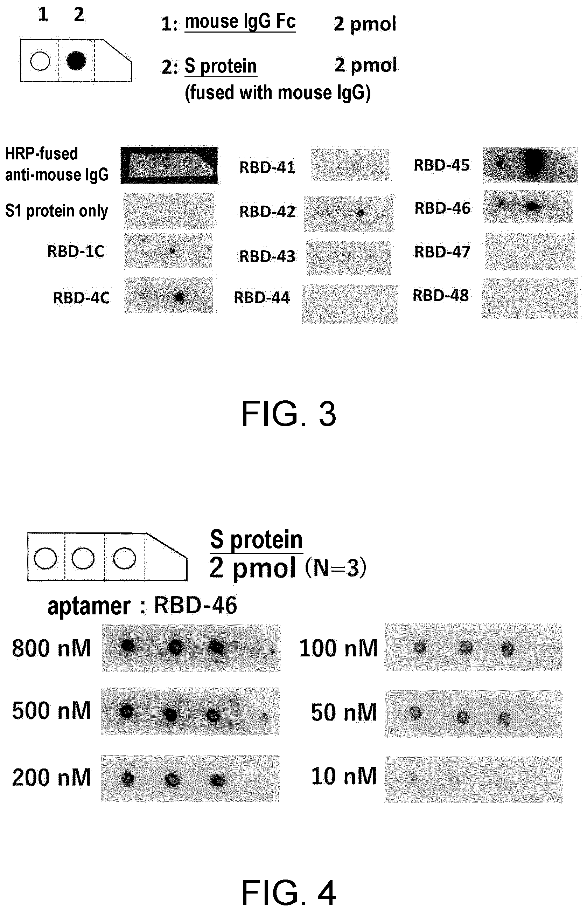

HRP chemiluminescent substrate was dropped onto the membrane and incubated at room temperature for 5 minutes. Chemiluminescence was then detected. The results are shown in FIG. 3.

As shown in FIG. 3, when RBD-45 or RBD-46 was used as the aptamer, stronger binding was observed than when other aptamers such as RBD-4C were used. Here, RBD-46, which showed stable and strong binding, was used in Reference Example 2.

### [Reference Example 2] Calculation of the dissociation constant of the novel coronavirus-binding aptamer

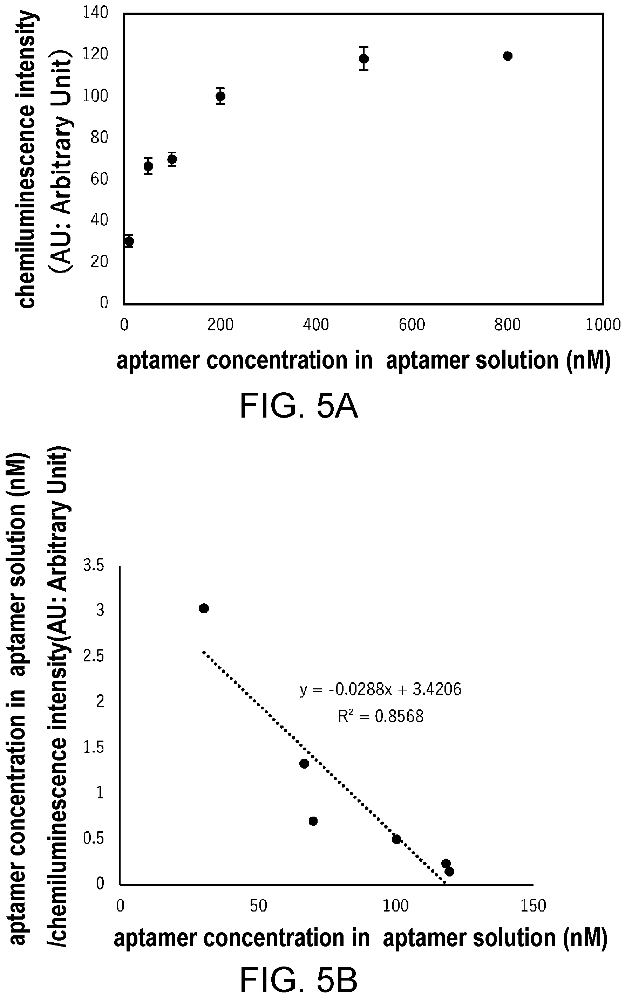

Aptamer blotting was performed in the same manner as in Reference Example 1, except that a 2 pmol specimen of SARS-CoV-2 S protein was used as the target protein and an aptamer solution containing 10 nM, 50 nM, 100 nM, 200 nM, 500 nM, and 800 nM RBD-46 was used. The results are shown in FIG. 4. FIG. 5A shows a graph in which the horizontal axis represents the aptamer concentration in the aptamer solution and the vertical axis represents the chemiluminescence intensity. FIG. 5B shows a graph in which the horizontal axis represents the aptamer concentration in the aptamer solution and the vertical axis represents the value obtained by dividing the aptamer concentration in the aptamer solution by the chemiluminescence intensity, ie, a Scatchard plot.

From the results shown in FIG. 4, FIG. 5A and FIG. 5B, the dissociation constant (K_{d}) of RBD-46 for the RBD of the SARS-CoV-2 S protein was calculated to be 34.7 nM.

### [Example 1] Detection method using gold nanoparticles

Prior to detection using gold nanoparticles, the following specimens were prepared.

### (PBS)

A stock solution of 10 times the concentration of phosphate buffer solution (PBS), "10x PBS Buffer" (manufactured by Nippon Gene Co., Ltd.), was diluted with ultrapure water to a concentration of 1x for use. PBS contains 137 mM NaCl, 8.1 mM Na₂HPO₄, 1.47 mM KH₂PO₄, and 2.68 mM KCl.

### (Preparation of SARS-CoV-2 S protein specimen)

SARS-CoV-2 (2019-nCoV) Spike RBD Recombinant protein (manufactured by Sino Biological) was diluted with PBS to concentrations of 1 µg/ml, 10 µg/ml, and 100 µg/ml.

### (Preparation of SARS-CoV-2 inactivated virus specimen)

The inactivated novel coronavirus (manufactured by RANDOX) was dialyzed using a Slide-A-Lyzer Dialysis Cassette (manufactured by PIERCE), and the buffer was replaced with PBS. The inactivated virus was diluted with PBS to concentrations of 10 pfu/ml, 100 pfu/ml, 1,000 pfu/ml, and 10,000 pfu/ml. The inactivated novel coronavirus concentration was calculated by converting 10,000 dC/ml to approximately 10,000 pfu/ml.

### (Preparation of inactivated influenza virus specimen)

For inactivated influenza virus (A/H1N1/PR/8/34), a 9.2 x 10⁵ pfu/ml culture solution was heat-treated at 100°C for 30 minutes, and then dialyzed using a Slide-A-Lyzer Dialysis Cassette (manufactured by PIERCE), and the buffer was replaced with PBS. The influenza virus was diluted with PBS to concentrations of 1,000 pfu/ml, 10,000 pfu/ml, and 100,000 pfu/ml.

### (Preparation of BSA solution)

As a control, fetal bovine serum (BSA) (manufactured by Roche) was dissolved in PBS to a concentration of 100 µg/ml.

### (Preparation of DNA aptamers)

Biotin-labeled RBD-46 (SEQ ID NO: 1) (1 µM) was diluted to 1 µM with 10 mM Tris-HCl (pH 7.5) containing 100 mM NaCl and 5 mM KCl, kept at 95° C. for 10 minutes and then cooled to 25° C. over 30 minutes to form a G-quadruplex structure.

### (Preparation of aptamer specimen)

Colloidal golds (containing gold nanoparticles with an average particle size of 70 nm) prepared by the citrate reduction method were diluted with ultrapure water to an OD₅₄₅ of 1, and 900 µL of this solution was mixed with 100 µL of 50 mM Tris-HCl (pH 9.5) and 100 µL of the biotin-labeled RBD-46 (1 µM) obtained by the above procedure, and allowed to stand at room temperature for 15 minutes. Then, 110 µL of 10 w/v % BSA solution (pH 9.5) was added and centrifuged at 8,000 g for 20 minutes, and the supernatant was removed. 1 ml of 50mM Tris-HCl of pH9.5 was added to the residue for suspension, and then centrifuged at 8,000 g for 20 minutes, and the operation of removing the supernatant was repeated twice. The residue was then dispersed by ultrasonic treatment to obtain gold nanoparticle-labeled RBD-46.

The obtained solution containing gold nanoparticle-labeled RBD-46 was measured for OD₅₄₅ using a spectrophotometer "NanoDrop ND-1000" (manufactured by Thermo Fisher Scientific), and diluted with 50 mM Tris-HCl (pH 9.5) to an OD₅₄₅ of 3. The prepared solution containing gold nanoparticle-labeled RBD-46 is also referred to as the "aptamer specimen."

### (Detection Method)

To 10 µL each of the SARS-CoV-2 S protein specimen, SARS-CoV-2 inactivated virus specimen, inactivated influenza virus specimen, control BSA solution, and PBS as a blank, obtained by the above procedure, 10 µL of the aptamer specimen obtained by the above procedure was added, and was allowed to stand at room temperature for 10 minutes. The final concentration of NaCl contained in the resulting reaction solution was 68.5 mM.

The color tone of each of the resulting reaction solutions was visually observed, and further, the turbidity was measured. The results are shown in Table 2.

[Table 2]

**Table 2**

| Specimen | Concentration | Color (visual observation) | OD₅₄₅ |
|---|---|---|---|
| Aptamer specimen | - | red | 3.10 |
| SARS-CoV-2 S protein | 0 µg/ml | colorless | 0.17 |
| | 1 µg/ml | light red | 0.20 |
| | 10 µg/ml | light red | 0.30 |
| | 100 µg/ml | red | 1.27 |
| SARS-CoV-2 inactivated virus | 0 pfu/ml | colorless | 0.17 |
| | 10 pfu/ml | colorless | 0.17 |
| | 100 pfu/ml | light red | 0.23 |
| | 1,000 pfu/ml | light red | 0.30 |
| | 10,000pfu/ml | red | 1.20 |
| Inactivated influenza virus | 0 pfu/ml | colorless | 0.17 |
| | 1,000 pfu/ml | colorless | 0.20 |
| | 10,000 pfu/ml | colorless | 0.21 |
| | 100,000 pfu/ml | colorless | 0.19 |
| BSA | 100 µg/ml | colorless | 0.21 |

As shown in Table 2, when the SARS-CoV-2 S protein specimen and the SARS-CoV-2 inactivated virus specimen were used, red color development was confirmed. This indicates that the gold nanoparticle-labeled RBD-46 bound to the SARS-CoV-2 S protein contained in the SARS-CoV-2 S protein specimen and to the SARS-CoV-2 S protein specimen contained in the SARS-CoV-2 inactivated virus specimen, i.e., the presence of the SARS-CoV-2 S protein, namely the analyte, was detected. The detection limits were estimated to be 1 µg/ml for the S protein specimen and 100 pfu/ml for the inactivated virus specimen.

On the other hand, when the BSA solution and the inactivated influenza virus specimen were used, no red color development was observed. This indicates that the gold nanoparticle-labeled RBD-46 did not bind to the substances contained in the BSA solution and the inactivated influenza virus specimen, i.e., the presence of the analyte, the SARS-CoV-2 S protein, could not be detected.

The above results show that the color tone changes depending on the concentration of the analyte, and that the concentration of the analyte may be qualitatively and quantitatively determined by measuring the turbidity. In other words, it has been demonstrated that the presence of a target analyte may be confirmed by the detection method according to one embodiment of the present invention.

Although the present invention has been described in detail and with reference to specific embodiments, it will be apparent to those skilled in the art that various changes and modifications may be made without departing from the spirit and scope of the present invention. This application is based on a Japanese patent application (Patent Application No. 2022-096870) filed on June 15, 2022, the contents of which are incorporated by reference into this application.

### Industrial Applicability

The detection method according to one embodiment of the present invention does not require special equipment such as antibodies, luminescent reagents, and luminescence detection devices used in immunoassays, and can easily detect an analyte in a short period of time, making it extremely useful as a simple detection method.

### Sequence Listing Free Text

SEQ ID NO: 1: Base sequence of RBD-46
SEQ ID NO: 2: Base sequence of RBD-1C
SEQ ID NO: 3: Base sequence of RBD-4C
SEQ ID NO: 4: Base sequence of RBD-41
SEQ ID NO: 5: Base sequence of RBD-42
SEQ ID NO: 6: Base sequence of RBD-43
SEQ ID NO: 7: Base sequence of RBD-44
SEQ ID NO: 8: Base sequence of RBD-45
SEQ ID NO: 9: Base sequence of RBD-47
SEQ ID NO: 10: Base sequence of RBD-48

## Claims

1. A method for detecting an analyte contained in a sample, the method comprising the following (1) and (2):
(1) mixing the sample and a salt with a gold-nanoparticle-labeled aptamer in which gold nanoparticles are bound to an aptamer capable of binding to the analyte; and
(2) judging that the sample contains the analyte in a case where there is no aggregation of the gold-nanoparticle-labeled aptamer in a mixture obtained by (1).

2. The method according to claim 1, wherein the salt is sodium chloride.

3. The method according to claim 1 or 2, wherein the aptamer is a polynucleotide consisting of a base sequence shown in SEQ ID NO:1.

4. The method according to claim 3, wherein the analyte is a SARS-CoV-2 spike protein.
